Europäisches Patentamt

European Patent Office

Office européen des brevets

(11) Publication number: **0 322 013 B1**

(12) **EUROPEAN PATENT SPECIFICATION**

(45) Date of publication of patent specification: **17.03.93**  (51) Int. Cl.⁵: **B01J 39/12**, B01J 27/16, B01J 20/28

(21) Application number: **88202769.1**

(22) Date of filing: **02.12.88**

(54) A process for the preparation of solid dispersions of acid salts of tetravalent metals having lamellar structure in an inert inorganic matrix, and the products so obtained.

(30) Priority: **22.12.87 IT 2315387**

(43) Date of publication of application:
**28.06.89 Bulletin 89/26**

(45) Publication of the grant of the patent:
**17.03.93 Bulletin 93/11**

(84) Designated Contracting States:
**AT BE CH DE ES FR GB GR LI LU NL SE**

(56) References cited:
**EP-A- 0 037 321**
**EP-A- 0 159 756**
**DE-A- 3 423 171**
**US-A- 4 444 904**

(73) Proprietor: **ENIRICERCHE S.p.A.**
**Corso Venezia 16**
**I-20121 Milan(IT)**

(72) Inventor: **Bellussi, Giuseppe**
**Via Alberto Scoto 44**
**I-29100 Piacenza(IT)**
Inventor: **Clerici, Mario Gabriele**
**Via Europa 34**
**I-20097 San Donato Milanese Milan(IT)**
Inventor: **Perego, Giovanni**
**Piazzale S. di Santarosa 4**
**I-20156 Milan(IT)**
Inventor: **Alberti, Giulio**
**via Ruggero Torelli 67**
**I-06100 Perugia(IT)**
Inventor: **Costantino, Umberto**
**Via Cortonese 3a**
**I-06100 Perugia(IT)**

(74) Representative: **Roggero, Sergio et al**
**Ing. Barzanò & Zanardo Milano S.p.A. Via Borgonuovo 10**
**I-20121 Milano (IT)**

Rank Xerox (UK) Business Services
(3.10/3.5x/3.0.1)

## Description

This invention relates to a process for the preparation of a solid dispersion of acid salts of tetravalent metals having lamellar structure in an inorganic matrix, as well as to the products so obtained which have large surface areas and acid catalytic activity.

The acid salts of tetravalent metals are members of a class of inorganic compounds among the most investigated during the last two decades because of their very good catalytic properties and of their ionic exchange capabilities (A. Clearfield - Inorganic Ion Exchange Materials - C.R.C. Press Boca Raton, FL USA, 1982). It is well known (see EP-A-0 094 919 that compounds of such class having lamellar structure can be delaminated by intercalation within their structure of an organic substance containing a proton acceptor group and of water till obtaining a stable colloidal water suspension of the intercalated acid salt.

It is also well known (see EP-A-0 202 710) that the colloidal water suspension of one of the best known members of such class, i.e. alphazirconium phosphate, can give rise to powders of the mentioned compound which have surface areas between 9 and 20 $m^2/g$.

It is clearly evident that the value of the surface area (and then of the catalytically active sites) obtained according to the prior art mentioned previously is too low to be employed for interesting industrial applications.

Now it has been surprisingly found that it is possible to increase in a remarkable way the surface area of acid salts having a lamellar structure, through the incorporation in a stable way of such compounds into a solid inert inorganic matrix that allows the laminas of acid salts to be kept at distances larger than the normal distances, so increasing the value of the active surface area. The present invention, therefore, provides a process for the production of a solid dispersion of lamellar-structure Zr alpha-phosphate, starting from Zr alpha-phosphate which has been delaminated by intercalation of an amine, comprising the steps of:

(a) Contacting an aqueous colloidal dispersion of said delaminated Zr alpha-phosphate with an aqueous slurry of colloidal silica, or with an aqueous solution of an oligosilicate produced by hydrolysis of a tetra-alkyl silicate in an aqueous solution of a tetra-alkylammonium hydroxide;

(b) Gelling the resultant mixture by addition of acetic acid thereto;

(c) Eluting the resultant gel with acetic acid to restore the H+-form of Zr phosphate;

(d) Washing the eluted gel with demineralized water, and

(e) Heat-treating the washed product at a temperature of from 80°C to 650°C to obtain a solid dispersion in which the weight ratio of Zr phosphate to silica is from 1:1 to 1:5.

If so desired, a drying step at 80°C is carried out after the gelling step (b) and a further drying step is carried out after the washing step (d).

The colloidal suspension is an aqueous slurry of colloidal silica, more particularly stabilized by ammonia addition, for instance the product commercially available as LUDOX from DuPont, and the water solution of the oligosilicate prepared by hydrolysis of a tetraalkylsilicate in a water solution of a tetraalkylammonium hydroxide is, in particular, the water solution of an oligosilicate having a silica content between 5 and 50 % by weight, said solution being obtained by hydrolysis of tetraethylsllicate in a water solution of tetrapropylammonium hydroxide (at a concentration between 10 and 30 %).

As regards the other conditions for the preparation of the water solution of oligosilicate, such conditions are as follows: the hydrolysis is carried out at a temperature between 2°C and 120°C, preferably between 5°C and 80°C, in a time between 0.2 and 24 hours.

As regards the weight ratios between the Zr-alpha-phosphate and the silica they can also be of 1/1 or greater, but preferably they are lower than 1/1 and, more particularly, they are more preferably less than 1/2, such ratios being especially preferably between 1/3 and 1/5.

The weight ratios between the Zr-alpha-phosphate and the aqueous slurry of colloidal silica in the preparation will be of such values as to give the ratios mentioned above in the final product. According to a preferred embodiment of the present invention, the gelled dispersion of the acid salt, before the washing operation and the possible drying, is eluted with a mineral or an organic acid, and in particular with acetic acid.

Furtherly $\alpha$-Zr(HPO$_4$)$_2$.H$_2$O, becomes easily delaminated through intercalation of n-propylamine, and in addition it has a very good heat stability (the condensation of the POH groups of the interlayer regions starts at temperatures higher than 400°C, while the disruption of the surface ≡POH groups occurs beyond 700°C) together with a high ionic exchange capacity (6.64 meq/g).

Some examples will be disclosed in the following with the aim of better illustrating the invention, but it is to be understood that the invention is not to be considered as limited to or by such examples.

Example 1

a) Preparation of delaminated Zr $\alpha$-phosphate

Crystalline zirconium phosphate having a lamellar structure was prepared according to the following procedure:

85 g of $ZrOCl_2.8H_2O$ were dissolved into 1.2 l of water. 80 ml of 40 % by weight HF was first added to the solution, and then 715 ml of 85 by weight phosphoric acid was added. The clear solution, placed in a plastic vessel, was heated on a water bath to 80°C and pre-humidified air was bubbled through such solution.

Full precipitation occurred in about 4 days. The crystals were separated from the solution, washed with distilled water till pH 4-4.5 and stored in a desiccator over phosphorus pentoxide.

5 g of zirconium alpha-phosphate ($\alpha$-$Zr(HPO_4)_2.H_2O$) prepared as disclosed above, whose crystals had average sizes of about 15 $\mu$m and surface area of 0.2 $m^2/g$ was suspended into 500 cc of water, and 250 cc of a water solution of 0.066 M n-propylamine was added. The addition was performed slowly and dropwise over about 4 hours and with vigorous stirring.

Keeping the mixture stirred for a further period of about 20 hours, a colloidal suspension of delaminated zirconium alpha-phosphate was obtained.

b) The precursor of the inert solid inorganic matrix

The precursor of the inert solid inorganic matrix was prepared according to the following procedure:

54.5 g of tetraethylsilicate was added with stirring to 50 g of a water solution of tetrapropylammonium hydroxide at the concentration of 19 % by weight and previously heated to about 40°C, and the whole mixture was kept stirring till a clear solution is obtained.

The solution was cooled to room temperature and demineralized water was added to make up for evaporation losses.

Thus a clear solution was obtained which was stable at room temperature.

c) The preparation of the catalyst

The colloidal suspension obtained in a) was stirred and the solution prepared in b) was added to the same till a homogeneous mixture was obtained. Such mixture was heated up to 80°C and acetic acid was added to the same dropwise and with stirring till gelification of the system. After drying at 80°C, the zirconium phosphate dispersed throughout the silica matrix was regenerated to the hydrogen form by elution with acetic acid and washing with demineralized water. The material was washed and dried, then it was heated up to 600°C in a stream of $N_2$ and was left for one hour in such conditions, then it was calcined in the air at the same temperature for 5 hours.

The solid so obtained was a dispersion of Zr alphaphosphate in silica at the concentration of 24 % by weight calculated with respect to the starting weight of $\alpha$-$Zr(HPO_4)_2.H_2O$, and it had a surface area of 458 $m^2/g$ (determined by the Sorpty 1750 Carlo Erba in $N_2$).

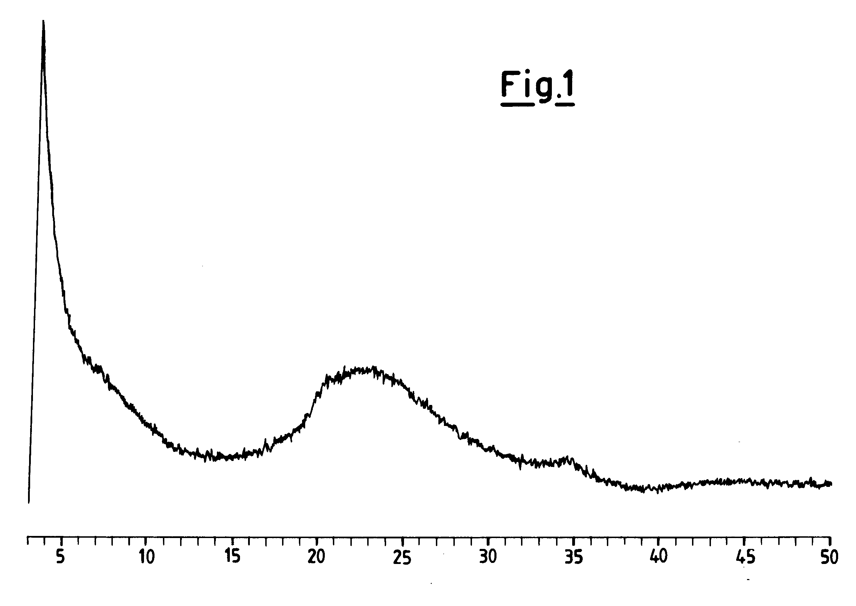

The X-ray diffraction pattern of the calcined product is shown in Figure 1. The almost complete disappearance of the crystalline character of the crystalline Zr alphaphosphate and the residual presence of the characteristic diffraction lines only, though widened to 2 $\theta \approx 20°$ and 2 $\theta \approx 34°$ (corresponding to planes of the crystalline lattice perpendicular to the surface of the lamina) is in agreement with the high dispersion of Zr phosphate throughout the silica matrix (almost at the level of an individual lamina). The bright diffused halo, centered at $2\theta \approx 22.5°$, corresponds to the diffraction of the glass silica matrix.

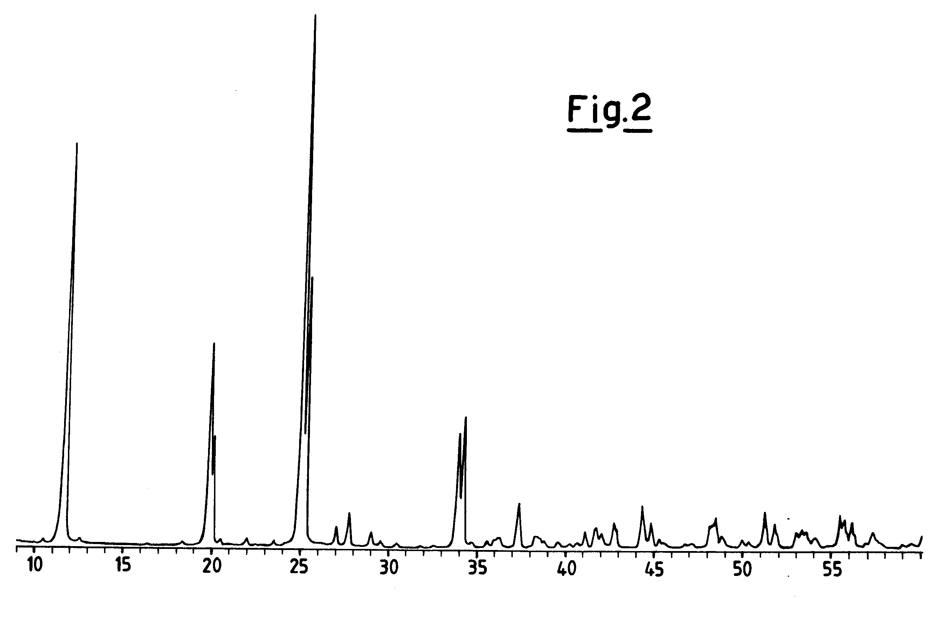

Figure 2 represents by comparison the X-ray diffraction pattern of crystalline Zr alpha-phosphate before delamination.

Example 2

a) The suspension of the delaminated Zr alpha-phosphate was prepared as disclosed in the paragraph a) of example 1.

b) The precursor of the inert solid inorganic matrix was made up of the colloidal silica product known as Ludox AS (30 % $SiO_2$), available from the DuPont.

c) 38.9 g of the water colloidal silica mentioned in the paragraph b) was added to the suspension obtained as disclosed in a) with stirring, and the suspension was kept stirring till a homogeneous mixture was obtained.

Such mixture was heated up to 80°C and acetic acid was added with stirring to the same dropwise, till gelification of the system. After drying at 80°C, the zirconium phosphate dispersed throughout the silica matrix was regenerated to the hydrogen form by elution with acetic acid and washing with demineralized water. The material washed and dried was then heated up to 600°C in a stream of $N_2$ and was kept for 1 hour in such conditions, then it was calcined in the air at the same temperature for 5 hours.

The solid so obtained was a 30% dispersion of Zr alpha-phosphate in silica gel, the percentage being calculated with respect to the starting weight of $\alpha$-$Zr(HPO_4)_2 \cdot H_2O$, and it had a surface area of 190 $m^2/g$ (determined by means of the Sorpty 1750 Carlo Erba in $N_2$).

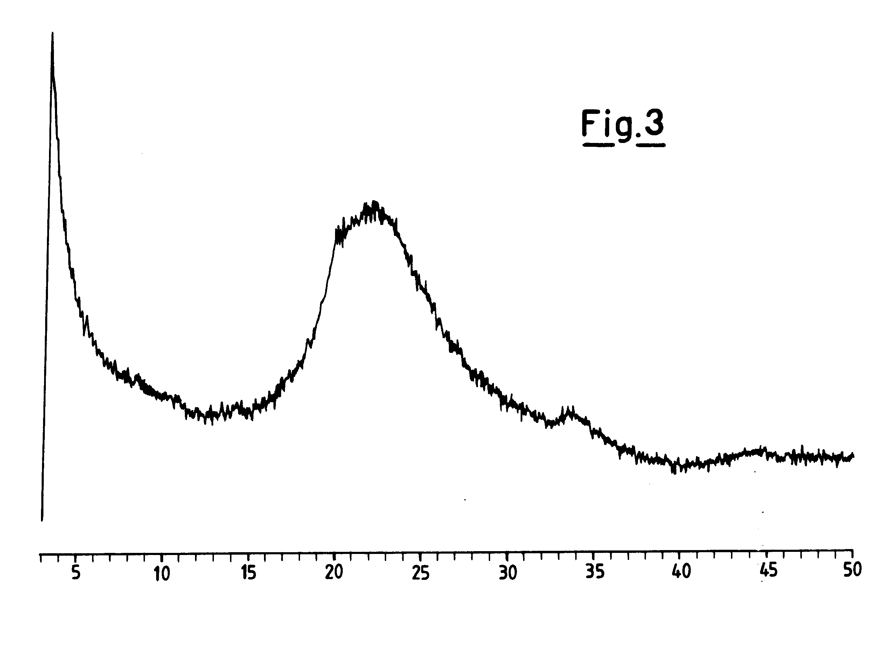

Figure 3 shows the X-ray diffraction pattern of the dispersion after calcination: the same considerations concerning Figure 1 hold true also for such diffraction pattern. The diffraction patterns reported in Figures 1-3 were obtained employing the $CuK_\alpha$ radiation; the abscissas represent angles $2\theta$, while the relative intensity was reported as the ordinates.

The materials disclosed above can be employed as cation exchangers or as catalysts. Some examples will be given in the following for illustrating their use as catalysts, and in the salt conversion through ionic exchange.

Example 3 (comparative)

Material consisting of non-delaminated crystalline Zr alpha-phosphate, prepared according to the disclosure given in paragraph a) of example 1.

Example 4 (comparative)

Material consisting of amorphous zirconium phosphate, prepared according to the procedure disclosed in the following publication: A. Clearfield, A. Oskarsson and C. Oskarsson, Ion Exch. Membr. 1, 9, 1972.

Example 5

Dehydration of cyclohexanol

Tests were carried out in a small reactor consisting of a glass pipe of inside diameter 0.8 cm, provided with a water condenser and a trap at -80°C for the more volatile products. Heating up to the desired temperature was carried out with a tubular oven provided with a device for temperature control. The catalyst, being a very fine powder, was first made into a tablet under pressure of 4 $t/cm^2$, then crushed and screened to 420 $\mu m$ - 250 $\mu m$ (35/60 mesh).

0.7 g of the catalyst was loaded into the reactor having a porous wall, together with a minimum layer of glass wool and finally 9 cm was loaded of an inert filler in order to favour evaporation and pre-heating of cyclohexanol. This compound was fed to the reactor by a metering pump of the syringe type (the Perfusor available from the Brown-Melsungen). During the test, a constant flow of nitrogen of about 0.9 l/hr was also kept. Temperature was determined with a thermocouple arranged inside the catalytic bed.

Before the reaction, the catalyst was previously treated at 350°C for 2 hours under a flow of dry nitrogen.

The products obtained after 30 minutes of reaction, collected in the trap, were weighed (with recovery better than 96 % of the material fed in), analyzed and quantitatively determined by the g.l.c. (with the method of the internal standard).

Their nature was confirmed by mass spectrometry by comparison with surely authentic samples.

Results of the catalytic tests, in terms of working temperature, conversion percentage of cyclohexanol and selectivity, are reported in the following.

4

### Dehydration of cyclohexanol (1)

| Cat. | T(°C) | Conv. of cyclohexanol (%) | Selectivity (%) | |
|---|---|---|---|---|
| | | | cyclohexene | methylcyclopentene |
| Ex.1 | 345 | 100 | 90 | 6 |
| Ex.2 | 347 | 100 | 82 | 12 |
| Ex.3 | 349 | 67 | 88 | - |
| Ex.4 | 350 | 7 | 90 | - |
| Ex.1 | 235 | 84 | 96 | 0.6 |
| Ex.2 | 234 | 70 | 95 | 0.5 |
| Ex.3 | 236 | 2.5 | 95 | - |
| Ex.4 | 235 | 0 | - | - |
| $SiO_2$ (2) | 233 | 0 | - | - |

(1) WHSV = 9.1

(2) Amorphous $SiO_2$ obtained from the precursor (b) of the inorganic matrix of example 1.

Example 6

1 g of the solid dispersion of zirconium alpha-phosphate in silica, prepared according to the specifications given in example 1, was immersed into 50 ml of a 0.100 M solution of CsCl that was kept under vigorous stirring. A decrease in the pH of the solution was immediately recorded, the pH passing from the initial value of 5.75 to the value of 2.05. To reestablish the initial pH value of 5.75 with the solid present in the suspension, 5.6 ml of a 0.100 M solution of CsOH was added. Accordingly, the ionic exchange capacity for the $Cs^+$ cation of the solid dispersion of zirconium alpha-phosphate in silica can be deduced to be of 0.56 meq/g under such conditions.

The same test was performed employing 1 g of silica prepared by gelification with acetic acid and next calcining at 600°C the precursor of the inert inorganic matrix prepared according to the specifications given in paragraph b) of example 1. In that case, the consumption of the 0.100 M CsOH solution turned out to be 0.2 ml. Accordingly, it can be deduced that the ion exchange capacity of the solid dispersion of zirconium alpha-phosphate in silica can be ascribed by 96% to the presence of acid protons of the $HPO_4$ groups of zirconium alpha-phosphate, and that such material, though dispersed throughout an inert inorganic matrix, is converted into a salt form by means of an ionic exchange reaction.

Example 7

1 g of the solid dispersion of zirconium alpha-phosphate in silica, prepared according to the specifications given in example 1, was immersed into 50 ml of a 0.100 M solution of copper acetate. The solution with the solid suspended was kept under constant stirring for two hours.

The analysis of the supernatant solution for determining the copper content gave 4.75 mmoles of $Cu^{+2}$ ions against 5.00 mmoles present at the start. It can be deduced that 1 g of solid dispersion of zirconium alpha-phosphate in silica shows under such conditions an ion exchange capacity for the $Cu^{+2}$ ion of 0.50 meq/g, and that zirconium phosphate dispersed throughout the silica was converted into the copper form.

**Claims**

**Claims for the following Contracting States : AT, BE, CH, DE, FR, GB, GR, LI, LU, NL, SE**

1. Process for the production of a solid dispersion of lamellar-structure Zr alpha-phosphate, starting from Zr alpha-phosphate which has been delaminated by intercalation of an amine, comprising the steps of:
   (a) Contacting an aqueous colloidal dispersion of said delaminated Zr alpha-phosphate with an aqueous slurry of colloidal silica, or with an aqueous solution of an oligosilicate produced by hydrolysis of a tetra-alkyl silicate in an aqueous solution of a tetra-alkylammonium hydroxide;
   (b) Gelling the resultant mixture by addition of acetic acid thereto;
   (c) Eluting the resultant gel with acetic acid to restore the H + -form of Zr phosphate;
   (d) Washing the eluted gel with demineralized water, and
   (e) Heat-treating the washed product at a temperature of from 80°C to 650°C to obtain a solid dispersion in which the weight ratio of Zr phosphate to silica is from 1:1 to 1:5.

2. Process according to Claim 1, wherein a drying step at 80°C is carried out after the gelling step (b) and a further drying step is carried out after the washing step (d).

3. Process according to Claim 1, wherein the aqueous slurry of colloidal silica is stabilized with ammonia.

4. Process according to Claim 1, wherein the aqueous solution of the oligosilicate is prepared by hydrolyzing tetraethylsilicate in an aqueous solution of tetrapropylammonium hydroxide, and contains from 5% to 50% by weight of silica.

5. Process according to Claim 4, wherein the hydrolysis is carried out at a temperature of from 2°C to 120°C and for a time of from 0,2 hours to 24 hours.

6. Process according to Claim 5, wherein the hydrolysis is carried out at a temperature of from 5°C to 80°C.

7. An ion-exchanger containing a solid dispersion of lamellar-structure Zr alpha-phosphate produced according to the process of Claim 1, as the active ion-exchanging component.

8. A catalyst containing a solid dispersion of lamellar-structure Zr alpha phosphate produced according to to the process of Claim 1, as the active catalyzing component.

9. A catalyst substrate containing a solid dispersion of lamellar-structure Zr alpha-phosphate produced according to the process of Claim 1.

**Claims for the following Contracting State : ES**

1. Process for the production of a solid dispersion of lamellar-structure Zr alpha-phosphate, starting from Zr alpha-phosphate which has been delaminated by intercalation of an amine, comprising the steps of:
   (a) Contacting an aqueous colloidal dispersion of said delaminated Zr alpha-phosphate with an aqueous slurry of colloidal silica, or with an aqueous solution of an oligosilicate produced by hydrolysis of a tetra-alkyl silicate in an aqueous solution of a tetra-alkylammonium hydroxide;
   (b) Gelling the resultant mixture by addition of acetic acid thereto;
   (c) Eluting the resultant gel with acetic acid to restore the H + -form of Zr phosphate;
   (d) Washing the eluted gel with demineralized water, and
   (e) Heat-treating the washed product at a temperature of from 80°C to 650°C to obtain a solid dispersion in which the weight ratio of Zr phosphate to silica is from 1:1 to 1:5.

2. Process according to Claim 1, wherein a drying step at 80°C is carried out after the gelling step (b) and a further drying step is carried out after the washing step (d).

3. Process according to Claim 1, wherein the aqueous slurry of colloidal silica is stabilized with ammonia.

4.  Process according to Claim 1, wherein the aqueous solution of the oligosilicate is prepared by hydrolyzing tetraethylsilicate in an aqueous solution of tetrapropylammonium hydroxide, and contains from 5% to 50% by weight of silica.

5.  Process according to Claim 4, wherein the hydrolysis is carried out at a temperature of from 2°C to 120°C and for a time of from 0,2 hours to 24 hours.

6.  Process according to Claim 5, wherein the hydrolysis is carried out at a temperature of from 5°C to 80°C.

**Patentansprüche**
**Patentansprüche für folgende Vertragsstaaten : AT, BE, CH, DE, FR, GB, GR, LI, LU, NL, SE**

1.  Verfahren zur Herstellung einer festen Dispersion von Zirconium-alpha-phosphat mit Lamellarstruktur, ausgehend von Zirconium-alpha-phosphat, das durch Einlegerung eines Amins delaminiert worden ist, welches Verfahren die folgenden Stufen umfaßt:
    (a) Inberührungbringen einer wässerigen kolloidalen Dispersion dieses delaminierten Zirconium-alpha-phosphats mit einer wäßrigen Aufschlämmung von kolloidalem Siliciumdioxid oder mit einer wäßrigen Lösung eines Oligosilicats, das durch Hydrolyse eines Tetraalkylsilicats in einer wäßrigen Lösung eines Tetraalkylammoniumhydroxids gebildet worden ist;
    (b) Gelieren des gebildeten Gemisches durch Zusetzen von Essigsäure;
    (c) Eluieren des gebildeten Gels mit Essigsäure zur Wiederherstellung der $H^+$-Form von Zirconiumphosphat;
    (d) Waschen des eluierten Gels mit entmineralisiertem Wasser und
    (e) Wärmebehandlung des gewaschenen Produktes bei einer Temperatur von 80°C bis 650°C zur Erzielung einer festen Dispersion, worin das Gewichtsverhältnis von Zirconiumphosphat zu Siliciumdioxid von 1:1 bis 1:5 beträgt.

2.  Verfahren nach Anspruch 1, worin nach der Gelierungsstufe (b) eine Trocknungsstufe bei 80° C ausgeführt wird und nach der Waschstufe (d) eine weitere Trocknungsstufe vorgenommen wird.

3.  Verfahren nach Anspruch 1, worin die wäßrige Aufschlämmung von kolloidalem Siliciumdioxid mit Ammoniak stabilisiert wird.

4.  Verfahren nach Anspruch 1, worin die wäßrige Lösung des Oligosilicats durch Hydrolysieren von Tetraethylsilicat in einer wäßrigen Lösung von Tetrapropylammoniumhydroxid bereitet wird und von 5 bis 50 Gew.-% Siliciumdioxid enthält.

5.  Verfahren nach Anspruch 4, worin die Hydrolyse bei einer Temperatur von 2°C bis 120° C und während einer Zeitdauer von 0,2 Stunden bis 24 Stunden ausgeführt wird.

6.  Verfahren nach Anspruch 5, worin die Hydrolyse bei einer Temperatur von 50°C bis 80°C ausgeführt wird.

7.  Ein Ionenaustauscher mit einem Gehalt an einer festen Dispersion von Zirconium-alpha-phosphat mit Lamellarstruktur, hergestellt nach dem Verfahren von Anspruch 1, als der wirksamen ionenaustauschenden Komponente.

8.  Ein Katalysator mit einem Gehalt an einer festen Dispersion von Zirconium-alpha-phosphat mit Lamellarstruktur, erhalten nach dem Verfahren von Anspruch 1, als der wirksamen katalysierenden Komponente.

9.  Ein Katalysatorsubstrat mit einem Gehalt an einer festen Dispersion von Zirconium-alpha-phosphat mit Lamellarstruktur, erhalten nach dem Verfahren von Anspruch 1.

**Patentansprüche für folgenden Vertragsstaat : ES**

1.  Verfahren zur Herstellung einer festen Dispersion von Zirconium-alpha-phosphat mit Lamellarstruktur, ausgehend von Zirconium-alpha-phosphat, das durch Einlagerung eines Amins delaminiert worden ist, welches Verfahren die folgenden Stufen umfaßt:
    (a) Inberührungbringen einer wässerigen kolloidalen Dispersion dieses delaminierten Zirconium-alpha-phosphats mit einer wäßrigen Aufschlämmung von kolloidalem Siliciumdioxid oder mit einer wäßrigen Lösung eines Oligosilicats, das durch Hydrolyse eines Tetraalkylsilicats in einer wäßrigen Lösung eines Tetraalkylammoniumhydroxids gebildet worden ist;
    (b) Gelieren des gebildeten Gemisches durch Zusetzen von Essigsäure;
    (c) Eluieren des gebildeten Gels mit Essigsäure zur Wiederherstellung der H$^+$-Form von Zirconiumphosphat;
    (d) Waschen des eluierten Gels mit entmineralisiertem Wasser und
    (e) Wärmebehandlung des gewaschenen Produktes bei einer Temperatur von 80°C bis 650°C zur Erzielung einer festen Dispersion, worin das Gewichtsverhältnis von Zirconiumphosphat zu Siliciumdioxid von 1:1 bis 1:5 beträgt.

2.  Verfahren nach Anspruch 1, worin nach der Gelierungsstufe (b) eine Trocknungsstufe bei 80°C ausgeführt wird und nach der Waschstufe (d) eine weitere Trocknungsstufe vorgenommen wird.

3.  Verfahren nach Anspruch 1, worin die wäßrige Aufschlämmung von kolloidalem Siliciumdioxid mit Ammoniak stabilisiert wird.

4.  Verfahren nach Anspruch 1, worin die wäßrige Lösung des Oligosilicats durch Hydrolysieren von Tetraethylsilicat in einer wäßrigen Lösung von Tetrapropylammoniumhydroxid bereitet wird und von 5 bis 50 Gew.-% Siliciumdioxid enthält.

5.  Verfahren nach Anspruch 4, worin die Hydrolyse bei einer Temperatur von 2°C bis 120°C und während einer Zeitdauer von 0,2 Stunden bis 24 Stunden ausgeführt wird.

6.  Verfahren nach Anspruch 5, worin die Hydrolyse bei einer Temperatur von 5°C bis 80°C ausgeführt wird.


**Revendications**

**Revendications pour les Etats contractants suivants : AT, BE, CH, DE, FR, GB, GR, LI, LU, NL, SE**

1.  Procédé de production d'une dispersion à l'état solide d'α-phosphate de zirconium à structure feuilletée, à partir d'α-phosphate de zirconium dont les feuillets ont été espacés par intercalation d'une amine, ledit procédé comprenant les étapes suivantes :
    a) mise en contact d'une dispersion aqueuse colloïdale dudit α-phosphate de zirconium à feuillets espacés avec une suspension aqueuse de silice colloïdale ou avec une solution aqueuse d'un oligosilicate, produite par hydrolyse d'un silicate de tétra-alkyle dans une solution aqueuse d'un hydroxyde de tétra-alkylammonium;
    b) gélification du mélange résultant par addition d'acide acétique;
    c) imprégnation du gel résultant avec de l'acide acétique, pour restaurer la forme H$^+$ du phosphate de zirconium;
    d) lavage du gel imprégné avec de l'eau déminéralisée; et
    e) traitement thermique du produit lavé, à une température de 80°C à 650°C, pour obtenir une dispersion à l'état solide, dans laquelle le rapport pondéral du phosphate de zirconium à la silice vaut de 1/1 à 1/5.

2.  Procédé conforme à la revendication 1, dans lequel on effectue une étape de séchage à 80°C après l'étape (b) de gélification, ainsi qu'une autre étape de séchage après l'étape (d) de lavage.

3.  Procédé conforme à la revendication 1, dans lequel la suspension aqueuse de silice colloïdale est stabilisée avec de l'ammoniaque.

4. Procédé conforme à la revendication 1, dans lequel la solution aqueuse d'oligosilicate est préparée par hydrolyse de silicate de tétra-éthyle dans une solution aqueuse d'hydroxyde de tétrapropylammonium et contient de 5 % à 50% en poids de silice.

5. Procédé conforme à la revendication 4, dans lequel l'hydrolyse est effectuée à une température de 2°C à 120°C et pendant un laps de temps de 0,2 heure à 24 heures.

6. Procédé conforme à la revendication 5, dans lequel l'hydrolyse est effectuée à une température de 5°C à 80°C.

7. Echangeur d'ions contenant, en tant que composant actif dans l'échange d'ions, une dispersion à l'état solide d'α-phosphate de zirconium à structure feuilletée, produite selon le procédé de la revendication 1.

8. Catalyseur contenant, en tant que composant catalytique actif, une dispersion à l'état solide d'α-phosphate de zirconium à structure feuilletée, produite selon le procédé de la revendication 1.

9. Support de catalyseur contenant une dispersion à l'état solide d'α-phosphate de zirconium à structure feuilletée, produite selon le procédé de la revendication 1.

**Revendications pour l'Etat contractant suivant : ES**

1. Procédé de production d'une dispersion à l'état solide d'α-phosphate de zirconium à structure feuilletée, à partir d'α-phosphate de zirconium dont les feuillets ont été espacés par intercalation d'une amine, ledit procédé comprenant les étapes suivantes :

a) mise en contact d'une dispersion aqueuse colloïdale dudit α-phosphate de zirconium à feuillets espacés avec une suspension aqueuse de silice colloïdale ou avec une solution aqueuse d'un oligosilicate, produite par hydrolyse d'un silicate de tétra-alkyle dans une solution aqueuse d'un hydroxyde de tétra-alkylammonium ;

b) gélification du mélange résultant par addition d'acide acétique;

c) imprégnation du gel résultant avec de l'acide acétique, pour restaurer la forme $H^+$ du phosphate de zirconium;

d) lavage du gel imprégné avec de l'eau déminéralisée; et

e) traitement thermique du produit lavé, à une température de 80°C à 650°C, pour obtenir une dispersion à l'état solide, dans laquelle le rapport pondéral du phosphate de zirconium à la silice vaut de 1/1 à 1/5.

2. Procédé conforme à la revendication 1, dans lequel on effectue une étape de séchage à 80°C après l'étape (b) de gélification, ainsi qu'une autre étape de séchage après l'étape (d) de lavage.

3. Procédé conforme à la revendication 1, dans lequel la suspension aqueuse de silice colloïdale est stabilisée avec de l'ammoniaque.

4. Procédé conforme à la revendication 1, dans lequel la solution aqueuse d'oligosilicate est préparée par hydrolyse de silicate de tétra-éthyle dans une solution aqueuse d'hydroxyde de tétrapropylammonium et contient de 5 % à 50% en poids de silice.

5. Procédé conforme à la revendication 4, dans lequel l'hydrolyse est effectuée à une température de 2°C à 120°C et pendant un laps de temps de 0,2 heure à 24 heures.

6. Procédé conforme à la revendication 5, dans lequel l'hydrolyse est effectuée à une température de 5°C à 80°C.

## Fig.1

EP 0 322 013 B1

# Fig.2

Fig.3